# EUROPEAN PATENT SPECIFICATION

(11) **EP 2 152 599 B1**
(45) Date of publication and mention of the grant of the patent: **15.02.2012**
(21) Application number: 08763866.4
(22) Date of filing: 16.05.2008
(51) Int. Cl.: B65D 47/20, B65D 47/18

(54) **MULTIDOSE DISPENSER FOR STERILE LIQUID PREPARATIONS**
MEHRFACHDOSISSPENDER FÜR STERILE FLÜSSIGE ZUBEREITUNGEN
DISTRIBUTEUR MULTIDOSE POUR DES PREPARATIONS LIQUIDES STERILES

(30) Priority: 17.05.2007 IT RM20070275
(43) Date of publication of application: 17.02.2010
(73) Proprietor: Federighi, Federigo, 56017 San Martino a Ulmiano (PA) (IT); Federighi, Mario, I-56123 Pisa (IT)
(72) Inventor: NARDI, Giancarlo, 56123 Pisa (IT)
(74) Representative: Banchetti, Marina
(86) International application number: PCT/IT2008/000328
(87) International publication number: WO 2008/142721

(56) References cited:
- EP-A- 0 172 711
- WO-A-2006/043295
- WO-A-2007/056233
- US-A- 4 099 651
- US-A- 5 971 357
- US-A1- 2003 173 380

## Description

The present invention concerns a multidose bottle for dispensing liquid preparations without preservatives. More specifically, the invention concerns a container specially designed for the sterile conservation of liquid products therein and their distribution in the form of drops, the sterility being maintained also after the first opening of the container, without the need to include preserving agents in the liquid product.

Many products, particularly but not exclusively in the medical field, are obtained and used in liquid form, such as solutions or mixtures of one or more liquid components with one or more dissolved solids; for their proper application, these liquids must be dispensed dropwise at the time of use. The most common example is that of liquid pharmaceutical products, both for systemic administration (oral drops) and more frequently for topical administration, such as on the nasal or oral mucosa, in the auricle of the ear and, above all, in the conjunctival sac. Topical ophthalmic products, such as eye-drops and artificial tears, also have the peculiarity that, besides needing to be administered in the form of drops, they must also be applied by the users on their own visual apparatus, and thus normally require the use of droppers with sufficiently elongated nozzles to facilitate as much as possible the actual application of the medication onto the surface of the cornea.

Other examples of liquid products that may require storage in vials or bottles and distribution in the form of drops are particular detergents or additives, also outside the pharmaceutical field, such as in the field of cosmetics or foodstuffs. These fields also have the problem of properly storing liquid products in sterile conditions and without altering their quality from one use to the next, and also in these fields, for particular applications, there may be required a dropwise dispensing of the product which may be as uniform and easily controllable as possible.

The conventional way of preventing or combating the microbial growth in liquid products to be used several times over after the first opening of the relative container consists of adding suitable preserving agents, antioxidants and antibacterial agents in the liquid product itself. The choice and concentration of the agents used must in any case be such to guarantee the necessary sterility within the envisaged conditions and expiry date of the product life, and at the same time must not alter the inherent characteristics of the liquid product.

More specifically, with reference to products for ophthalmic use that are packaged in multidose bottles of flexible material, the initial sterilization of the product takes place during the bottling phase, after which the bottle is sealed and packaged, and arrives in the consumer's hands in sterile form. When the bottle is first opened, the product would still be in a sterile condition, also without the use of preserving agents. On first use, by gently squeezing the bottle some liquid is forced out and made to pass through the dropper nozzle to reach the user. When the pressure exerted on the bottle ceases, the bottle stops dispensing the product; however, due to the elasticity of the material used, as soon as the pressure on the bottle ceases, the bottle returns to its original shape and reintegrates the volume of liquid dispensed by sucking in air from the outside environment through its nozzle. This air, which is potentially polluted, contaminates the remaining liquid in the bottle by starting an oxidative process on the volume of product left inside. This contaminating activity exerted by the outside air is normally prevented by using preservatives or antioxidants: without these, the pharmaceutical product could only be used once because the remaining liquid would become irreparably altered and unusable. All this would be considerably worsened if, during administration, the dropper came into contact with the user's body, which may potentially be contaminating.

Unless an alternative administration method is used - something which can only be economically justifiable in certain cases - namely the use of single dose packages containing a quantity of product for just one application, which do not require any preservatives, in the other cases the use of preservatives and antioxidants in multidose bottles intended to contain sterile preparations is made essential in view of a number of features that the product has to maintain over time, and especially after the container is first opened. These features include the capacity to maintain the liquid in sterile conditions after the bottle is first opened, and also when non-sterile air is sucked back in to replace the liquid used and, in addition, in the case of accidental contact between the dropper and parts of the user's body that may be polluting. Such features also include the capacity to resist oxidation due to contact with the outside air that increasingly replaces the volume of liquid used, and this throughout the useful life of the product once the bottle is first opened.

The addition of preservatives to liquid products for ophthalmic use, although admitted in the pharmacopoeia and commonly accepted, is not devoid of a series of drawbacks, however. These include, in particular, the possibility of causing burning sensations or inflammatory or allergic reactions as well as, above all, the proven cytotoxicity of these products. For example, it is well-known that some preservatives used for ophthalmic products have a toxic effect on the goblet cells of the cornea, so that their prolonged use could cause more problems than the ones they can actually help to overcome.

One possible strategy to avoid using preservatives in multidose bottles of liquid products is to provide one or more antibacterial filters on the liquid pathway towards the exit point in the bottle, in particular, filters with openings or pores of about 0.2 micron in diameter, which mechanically prevent any micro-organisms from passing through them, while not preventing the flow of liquid out. Examples of devices using such solution are disclosed in the international patent application publ. No. WO 90/05110 (Eye Research Institute of Retina Foundation), concerning a multidose bottle equipped with a pair of filters, one hydrophilic and the other, more downstream, water-repellent, placed along the liquid outward path, and in the US patent 5105993 (La Haye Laboratories), employing an antibacterial filter placed inside the dropper nozzle, which also employing a further filter placed on a venting port in the bottle. It is obvious that the adoption of an antibacterial filter, does not avoid that the section of the dropper duct lying downstream of the filter is still exposed to contamination by the external environment.

Still in the specific field of pharmaceutical products for topical use, U.S. patent No. 5,232,687 (Ursapharm) proposes adopting another, alternative or complementary, device in order to guarantee the sterility of a liquid product for topical administration without resorting to preservatives: the use of a solid substance with a germicide action like silver. The device proposed to this aim is in practise a small reciprocating pump spray dispensing metered amounts of liquid product. During the passage through a dispensing chamber, the product must come into close contact with a silver-based element with germicide functions. In this case, any inflowing of air inside the product container is not allowed by the fact that the small pump is made airtight.

Another commercially available device for dropwise dispensing liquid pharmaceutical products not requiring the use of preservatives is described in U.S. patent No. 6,336,571 (Laboratoires Théa), where a container with flexible bellow-shaped walls is used, protected by an external rigid shell, that is removable in order to allow access to the bellow-shaped bottle containing the liquid during the administration. Also in this case, the necessary aseptic conditions are provided by an antibacterial filter placed along the product outflow path.

A further solution for manufacturing multidose dispenser bottles for liquid products without preservatives is described in European patent application EP-A-1319606 (Nihon Tenganyaku Co. and Taisei Kako Co.), which concerns a double-walled container with an external flexible wall and an internal collapsible one, in which the manually exerted pressure on the external container is transmitted to the internal wall thereby forcing the liquid out during administration. Then, due to the presence of a vent hole on the external container, the latter can elastically return to its original shape by sucking air into the gap between the external wall and internal collapsible wall. This air does not come into contact with the product enclosed inside the internal container, thus preventing any possible contamination to the product itself.

The outflow of the product from the bottle occurs through a check valve that does not allow the liquid to come back into the container, but downstream of this check valve the liquid still passes through an antimicrobial filter before leaving the container. Also in this case, therefore, the device includes a space between the check valve and the dropper tip on which the filter is located, wherein some stagnant liquid, which is not excluded from contact with the outside air, is left even if it should be protected from the entrance of micro-organisms.

In general, the known devices envisaging the presence of check valves on the liquid outflow duct from the bottle still have sections downstream of the check valve, which may particularly include the dropper nozzle, where stagnant liquid remains after administration and where contaminating air can enter from the outside.

A dispenser bottle for products in drops without preservatives, provided with a check valve realised directly onto the dropper nozzle, operating on the dropper tip itself and automatically activated at the end of the pressure exerted by user on the container body, is described in the international patent publ. No . WO2006/043295 (of the same applicants). The device includes a dropper nozzle designed with a pin plug having a frusto-conical shape, complementary to the nozzle shape, wherein, however, the valve element having the plug function is fixed with respect to the bottle body, and the element having the function of the corresponding seat, namely the nozzle, is displaceable. The terminal lower part of nozzle, where the latter joints the bottle thereby blocking it in an airtight manner, includes an annular membrane expansion chamber sufficiently flexible to allow the dropper nozzle to extend forward with respect to the fixed plug when the pressure on the liquid contained in the bottle increases as a result of a pressure exerted on the outside of the bottle. The slight forward displacement of the nozzle with respect to the fixed plug opens around said plug a gap sufficiently wide to allow the passage of the liquid to be dropwise dispensing, which flows out from the nozzle tip. As soon as the externally applied pressure ceases , the annular membrane contracts again, automatically causing the nozzle to draw back with respect to the fixed pin plug.

In the disclosed device, the closure of the dropper after administration is automatic and, above all, it occurs exactly on the dropper tip, without leaving liquid residues in intermediate positions within the dispenser. However, the device that can be produced according to the teaching of the said document suffers from some manufacturing complexity, firstly because the bottle has to be produced with a double wall, with a collapsible container for the product inside and a bottle made of flexible material outside, with an air gap there between.

Based on such prior art, therefore an object of the present invention is to provide a dispenser device for products in drops which, while not requiring the addition of preservatives in the product, is able to protect the product from the entrance of polluting agents in the dropper nozzle, constituting the terminal supplying element, thus preventing not only air from entering from outside, but also the presence of inactive volumes and stagnation zones within the device, so as to avoid that the dropper interior remains not protected and moist with product.

Besides the pin plug valve described above, another kind of device for stopping and regulating the product flow from a dispenser of the same kind as those here at issue which allows to obtain the closure of the dispensing conduit in the most external point of the nozzle, with no zones of liquid stagnation or zones where potentially polluted air may return or stay, after the supply, is the unidirectional flow valve often refer to as sleeve valve. This is normally made of a flexible sleeve made of an elastomeric material or a material resiliently stretchable, such as silicone rubber, which surrounds a rigid tubular projection, having a generally cylindrical or conical shape (nozzle), directly communicating with the bottle interior on which some apertures are present for the product to be administered. The apertures are normally two or more radially extending ports provided on the rigid projection; said ports are generally closed by the resilient sleeve, but they allow liquid to flow outward from the interior of the container when a sufficient pressure is exerted on it so as to counter the radial pressure of the resilient sleeve, the latter being normally pre-loaded and adhering to the tubular projection forming the nozzle. As soon as the dispensing pressure is released, the sleeve resiliently returns to close the ports for the liquid passage, and inactive zones around the sleeve are left where some non-dispensed liquid may remain or air or polluted material from the exterior may return.

An example of a dispensing device suitable for medicaments or other liquid products which must be preserved from contamination or protected from oxidation in spite of the repeated and protracted use is disclosed in the European patent application EP-A-172711 (The Boots Company), concerning a collapsible container for liquid medicaments or similar, where in the product is dispensed through a resilient sleeve valve cooperating with the substantially cylindrical nozzle, which is in communication with the container interior and is provided with suitable radial apertures. Since the sleeve valve system alone cannot guarantee against abnormal accidental pressures excreted on the bottle, which would result in undesired outflow of liquid, the device is also provided with a closure element, consisting of a cap (in the case shown, a snap cap) which surrounds and encloses the dispensing nozzle and presses, with an internal projection of the cap itself, the terminal portion of the dispensing nozzle, close to the tip thereof.

The device concerned, as well as other known devices, where a liquid supply occurs through a resilient sleeve valve are combined, in order to prevent the accidental leakages of the liquid contained in the bottle, with closure systems (caps or capsules) wherein a rigid element exerts a pressure against the nozzle tip where the resilient sleeve is present, which represents the spontaneous closure zone of the sleeve system.

US 2003/0173380 and US 4 099 651 disclose further examples of dispensing devices.

In view of the foregoing, according to the present invention there has been devised a bottle or container for liquid products to be administered dropwise which is particularly suitable for the packaging of multidose pharmaceutical liquid products without preservatives, equipped with a dispensing system with unidirectional flow sleeve valve, which is suitable to insure the outmost asepticity to the product contained therein, having an improved performance in comparison with the known devices of the same kind. Actually an improved performance has been obtained both from the point of view of a reliable size control of the dispensed drops and from that of preventing from the possible accidental outflow of the product, due to an abnormal mechanical pressure on the bottle.

The regularity in volume of each drop of dispensed product, as well as the ability not to give rise to sprays or to continuous jets of product when the bottle is too energetically squeezed, are extremely important aspects in the pharmaceutical field. This is the case, for instance, of the drop-wise dosing of a product like an antibiotic collyrium or an oral paediatric solution, where an irregular size of the drops results in uncontrollable variations of the administered dosage. On the other hand, as already noted, the possibility of accidental outflow of product brings about, in addition to a loss of product, the pollution of the external zone surrounding the nozzle, with some liquid stagnating, for instance, between the nozzle and the external capsule.

In order to prevent the occurrence of similar shortcomings, the present invention proposes, as a first measure, to realize the rigid projection making out the dispensing nozzle, on which the elastomeric material sleeve is to be placed, with at least two subsequent portions having different conicity: namely, a first portion more tapered (i.e., with a greater conicity) closer to the body of the bottle and bearing the ports for the passage of the product contained in the bottle, and a second portion in a terminal position, with a lesser conicity, making out the proper tip of the dropper nozzle, the resilient sleeve extending in such a way as to cover both said portions. The portion with lesser conicity, which makes out the elongated dropper tip, is shaped in such a way as to never come into contact with other elements of the same device, so that the elastomeric material sleeve does not undergo any disturbance in the point where it is more delicate. The intermediate portion with greater conicity, i.e. the portion where the ports for the passage of product from the bottle interior are provided, is also the portion where the internal closure element of the cap abuts. Thus, the contact between the cap and the elastomeric sleeve does occur on the dropper tip, rather in the more tapered intermediate section, where the sleeve is more resistant to stresses that might tear it or deform it, and where a closing action on the ports for the liquid passage is directly exerted, such ports actually being located in that position in the dropper.

Therefore, the present invention specifically provides a dispenser for liquid preparations without preservatives, comprising a container body, a dropper assembly, and a closure capsule suitable to be coupled with said dropper assembly, wherein the dropper assembly in turn comprises a closure element for the container body ending with a rigid dropper nozzle provided with one or more transversal passage ports, communicating with the interior of said container body covered with a resiliently deformable tubular sleeve, cooperating with said ports for intercepting a liquid preparation contained in said dispenser, characterized in that the said dropper nozzle comprises at least two axially subsequent portions having different conicity: a first portion with smaller conicity making out the distal of said dropper nozzle and a second portion with greater conicity on which said one or more ports are provided, and in that said closure capsule comprises an internal projection that comes into contact, when the capsule is closed, with said resiliently deformable tubular sleeve at a position corresponding to the second portion of the dropper nozzle with greater conicity, thereby blocking the possible passage of the liquid preparation through the said two or more ports.

According to some preferred embodiments of the invention, the dropper assembly further comprises a protective tubular element coaxial with the dropper nozzle, which extends outside the tubular sleeve in such a way as to surround it for most of its length, so as to protect it accidental contacts with the exterior when the closure capsule is not present, that is when the package is open and the device is in use.

Preferably, the said resiliently deformable tubular sleeve (or sheath) comprises a first portion and a second axially subsequent portion, corresponding to said first and second portion of the dispensing nozzle. Also these portions, following the shape of the nozzle on which the sleeve is mounted, may have two sections with different conicity, and may also have different thickness, the smaller thickness being possibly reserved to the sleeve portion corresponding to the distal end of the dropper. As it will be clear with reference to the enclosed drawings, different tapering degree of two sections of dispensing nozzle causes the liquid dispensed by the device, which must overcome the pressure exerted by the sleeve which becomes radially dilated, once the first section with greater conicity is passed, flows much more easily in the nozzle portion with smaller conicity. As a result, the dispensing function is very well regulated as a portion of the pressure exerted by the user's fingers on the dispenser body.

According to some preferred embodiments of the invention, the resiliency deformable tubular sleeve comprises, at its end facing the container body, a third portion having a greater thickness than the previous ones, ending in an annular projection. The latter is coupled with the end of the cited protective tubular element facing towards the container body, so that the protective tubular element blocks the resilient sleeve in its seat, preventing it from slipping off the dispensing nozzle.

Preferably, the tubular protective element is also provided with an intermediate flange which is fixedly blocked on said closure element of the container body, in such a way that the same tubular protective element cannot slip off the dispenser when the latter is in use.

As noted, the liquid contained in the multidose dispenser according to the invention, under the effect of a manual pressure exerted on the container body, passes through the said two or more ports provided on the first portion with the greater conicity of the dispensing nozzle and flows within the resilient sleeve thereby reaching the dispensing nozzle. In order to obtain a distribution as homogeneous as possible of the dispensed liquid said portions are three in number, placed at 120° one from the other around the periphery of the nozzle.

As the other devices of the prior art the dispenser according to the invention may have a single-walled container body of the bottle type, made of flexible resilient material. As an alternative, it may be of the double-walled type with a collapsible internal container and an air gap place there between, or else it may be of the single-walled collapsible tube type. In the latter case, the tube may be provided with a conventionally shaped cap, or with cap allowing it to be placed upside down ("top-down" tube container).

The preferred embodiment, both in view of its practicality of use and in view of its cheapness and ease of realization at an industrial level, is the single-walled type bottle made of resiliently flexible material. In this case, the presence of the double-walled container with the internal air gap may be avoided thanks to the use of a venting valve with hydrophobic membrane for the passage of the gases only from and into the bottle, of the same type of the membranes available on the market with the commercial name Gore^{™}. Preferably, the hydrophobic membrane has a porosity of 0.2 µm, so as to function also as mechanical filter against the micro-organisms possibly carried with the air sucked in through the valve in replacement of the volume of liquid product dispensed.

The venting valve with hydrophobic membrane has a substantially cylindrical shape and it is housed, according to a preferred embodiment of the invention, in a suitable seat within the closure element of the container body, which is included in the dropper assembly.

The dispenser according to the invention is also completed by an external closure capsule suitable to couple with the dropper assembly described above, and which preferably comprises a screw cap suitable to protect the said dropper nozzle and an external knurled overcap outside the screw cap and connected to it. The combination of screw cap and overcap creates a safety closure system that prevents accidental opening of the bottle, like the kind of devices normally used on pharmaceutical bottles to avoid any improper use by children (child-proof devices). In addition, the capsule envisages a part frangible on first opening the bottle acting as a guarantee seal (tamper-evident device).

The specific features of the present invention, as well as its advantages and corresponding operating modalities, will be more evident further on with reference to a specific embodiment of the invention, illustrated merely for exemplary purposes in the attached drawings, wherein:
Figure 1 shows a longitudinal cross-section of a multidose dispenser for liquid preparations according to the present invention, particularly designed for dispensing pharmaceutical products drop by drop without using preservatives, and complete with all its closure elements;
Figure 2 shows a longitudinal cross-section of the same bottle of Figure 1, without the external closure capsule;
Figure 3 shows a longitudinal cross-section of the closure capsule of the same dispenser;
Figures 4A and 4B respectively show a longitudinal cross-sectional view and a lateral view of the closure element of the container body, which is the main element of the dropper assembly of the dispenser of the previous figures;
Figure 4C is a top plan view of the same element of Figures 4A and 4B;
Figure 4D shows a perspective view of the same element of Figures 4A-4C;
Figures 5A, 5B, and 5C, respectively show a longitudinal cross-sectional view, a lateral view and a perspective view of the tubular sleeve of the dropper assembly of the dispenser shown in the previous figures;
Figures 6A, 6B, and 6C, respectively show a longitudinal cross-sectional, a top plan view and a perspective view of the tubular protective element of the dropper assembly of the dispenser shown in the previous figures;
Figures 7A, and 7B, respectively show a longitudinal cross-sectional view and a perspective view of the venting valve element with hydrophobic membrane (Gore^{™}) of the dropper assembly of the dispenser shown in the previous figures;
Figure 8A shows a longitudinal cross-sectional view of the cap that constitutes an element of the closure capsule of the dispenser of Figure 1;
Figures 8B, and 8C, respectively show a top plan view and a perspective view of the same cap of Figure 8A;
Figure 9A shows a longitudinal cross-sectional view of the overcap constituting an element of the closure capsule of Figure 1;
Figures 9B and 9C respectively show a top plan view and a perspective view of the same overcap of Figure 9A;
Figure 10 shows a schematic view, in cross-section and partially broken away, of the dispenser of Figure 1, with the cap constituting an element of the closure capsule but without the overcap; and
Figure 11 shows schematic view in longitudinal cross-section and partially broken away of the same dispenser of Figure 10, without the cap.

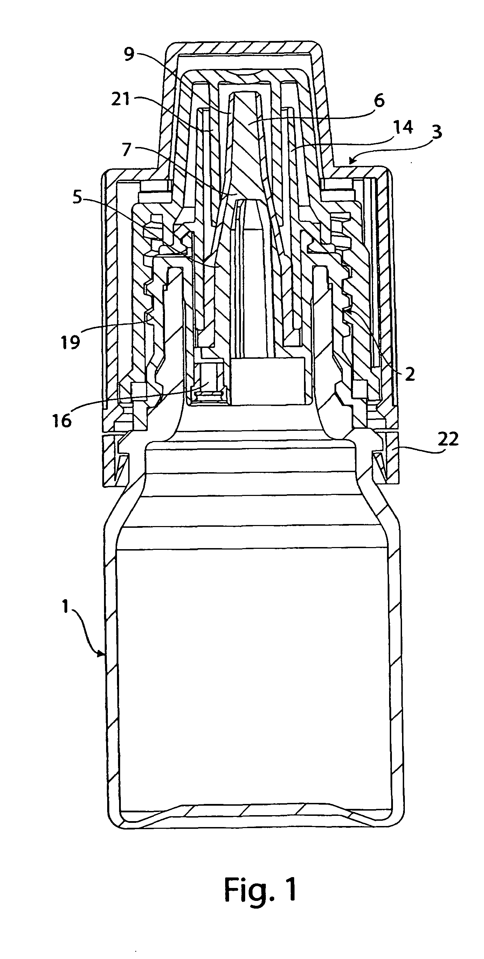

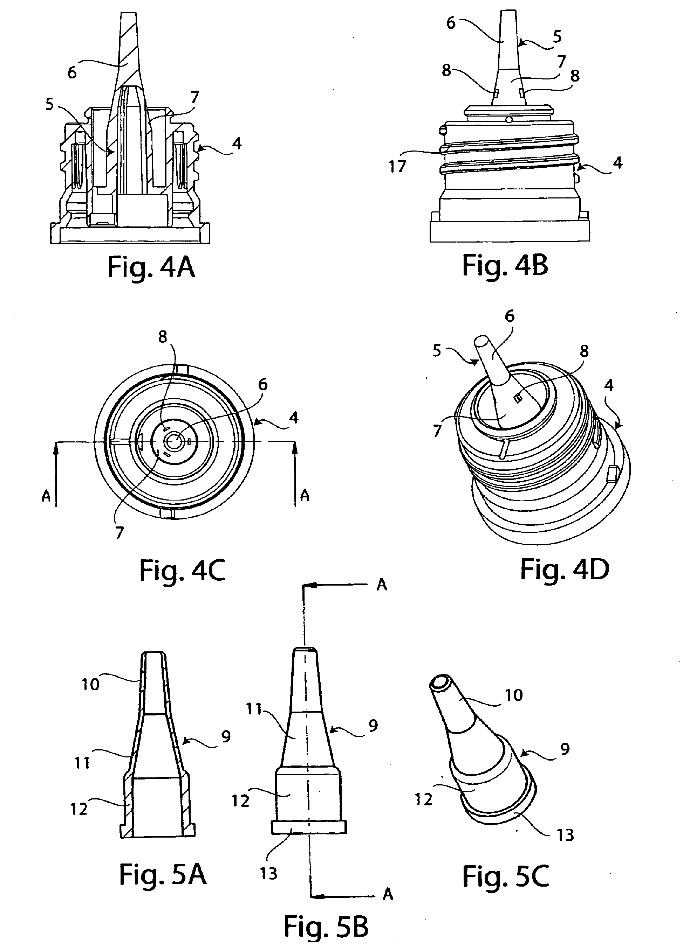

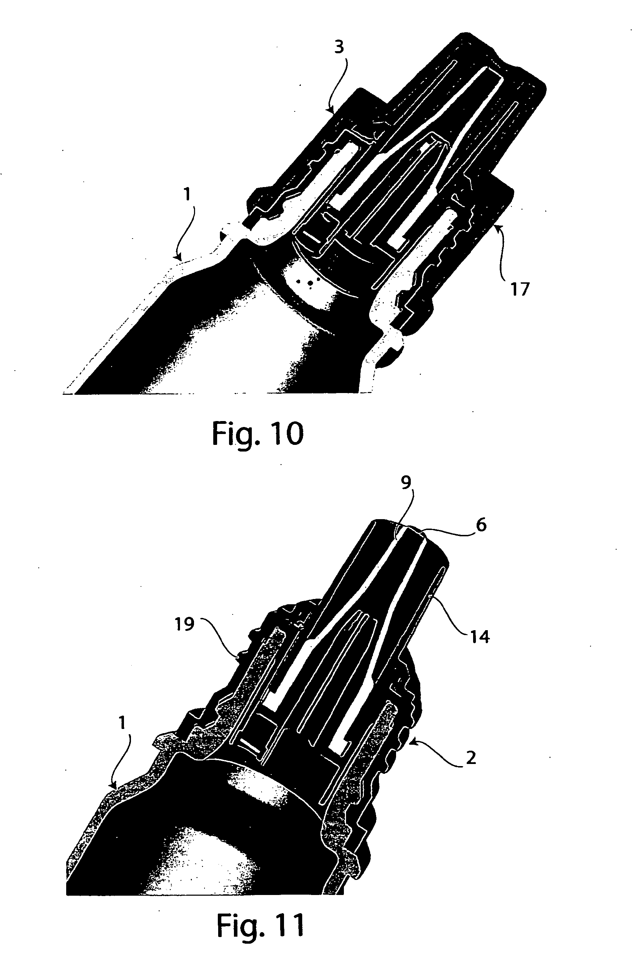

As it is shown in the overall views of Figures 1, 2 and 3, as well as in the schematic drawings of Figures 10 and 11, the illustrated embodiment of dispensing device for liquid products according to the invention consists of a container body (1) closed on top by a dropper assembly (2) and completed by a closing capsule (3). The dropper assembly (2), which is also shown in greater detail in Figures 4A-D, 5A-C, 6A-C and 7A-B, comprises in turn a closure element (4), individually shown in Figures 4A-B, consisting in a single piece of moulded plastic material. The latter ends in a rigid dropper nozzle (5), having a frusto-conical shape with a lesser conicity in its terminal end portion (6) and more tapered in the intermediate portion (7). The terminal portion (6) is full, while the intermediate portion (7) is internally hollow and communicates with the interior of the container body (1), being provided with three ports (8) radially placed at 120° around its periphery.

The second main element of the dropper assembly (2) consist of a tubular sleeve (9) made of elastomeric material, in particular silicone rubber, surrounding the dropper nozzle (5) both at the level of the terminal portion (6) at the level of the intermediate portion (7) with grater conicity. Besides Figures 1-3, and 10, 11, the tubular sleeve (9) is individually shown in Figures 5A-C.

In the embodiment shown herein, the resilient tubular sleeve (9) comprises three separate portions with different conicity and thickness, of which the terminal portion (10) having a smaller thickness is the one covering the terminal portion (6) of the dropper nozzle (5), the intermediate portion (11) is the one covering the intermediate portion, with greater conicity, of the dropper nozzle and the third portion (12) with a greater thickness than the two previous portions, is located close to the mouth of the dropper nozzle (5), and comprises an annular projection (13) intended to block the tubular sleeve (9) on the dropper nozzle (5), in such a way as to prevent it from slipping off. Said block is carried out with the cooperation with the tubular protective element (14), as it is more clearly shown in Figure 11.

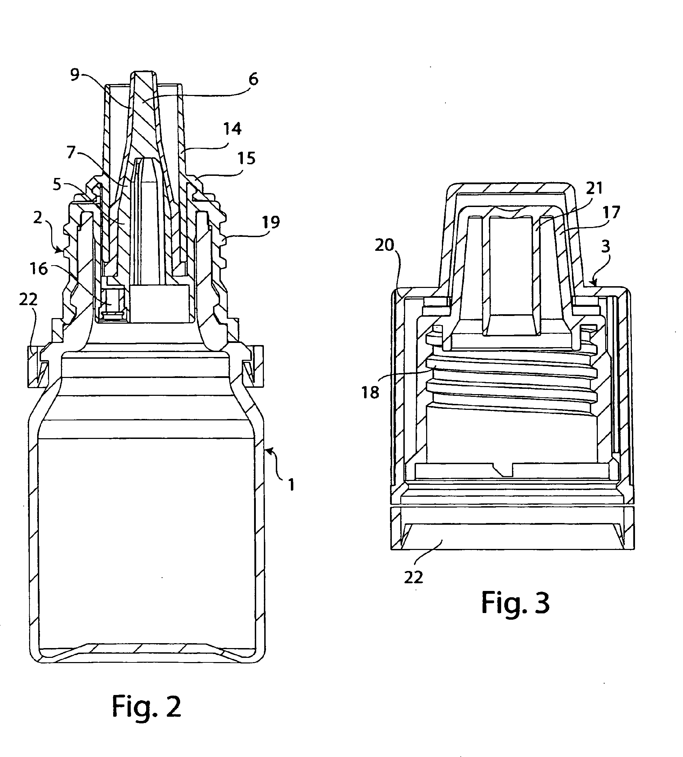

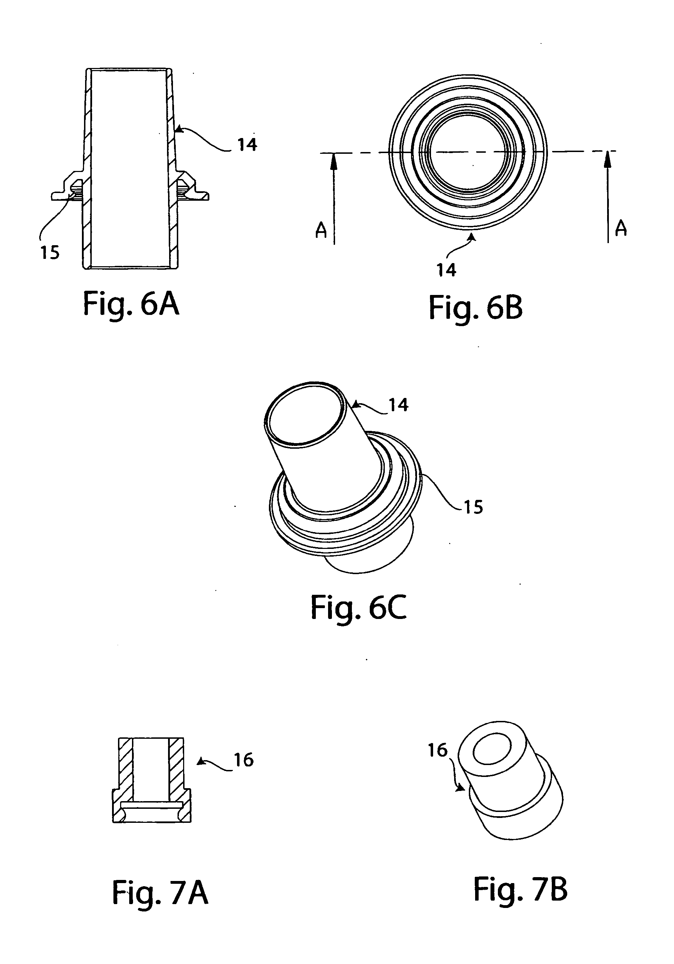

The tubular protective element (14), individually shown in Figures 6A-C , is in practise a roughly cylindrical component which is placed around the dropper nozzle (5), or better around the tubular sleeve (9), in order to protect it from accidental contacts during the use of the dispenser. As it is more clearly shown in Figures 2 and 11, the presence of such rigid element around the dropper (5) protects the elastomeric sleeve (9) from any contact, so that its working, and therefore the accuracy and uniformity of the dosage of product drops through it is no disturbed by accidental mechanical interference.

As it is shown both in Figures 10 and 11 and in Figure 2, the tubular protective element (14) is provided with the flange (15) in an intermediate position along its length, by which the said element fixedly engages the closure element (4) of the dropper assembly (2).

The fourth component present in the dropper assembly (2) shown consist of a venting valve (16) with hydrophobic membrane, individually shown in the Figures 7A and 7B, which in the embodiment shown consists of a Gore^{™} valve, provided in its port with a membrane of 0,2 µm of porosity which allows the passage of air through it but not the passage of liquid, and therefore allows the bottle to be refilled with the volume of air equal to the volume dispensed product, while maintaining the total sterility of the product contained and of the environment coming into contact with said product.

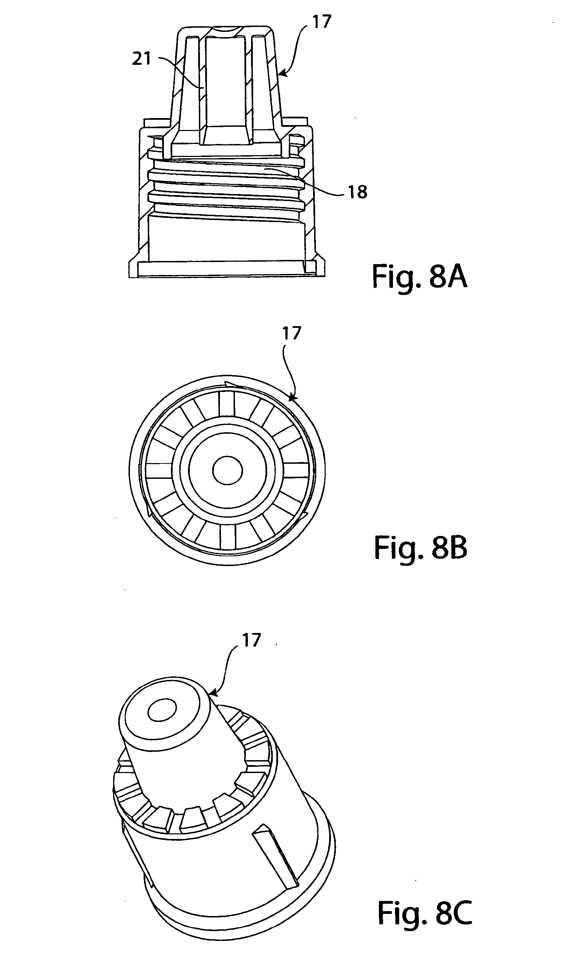

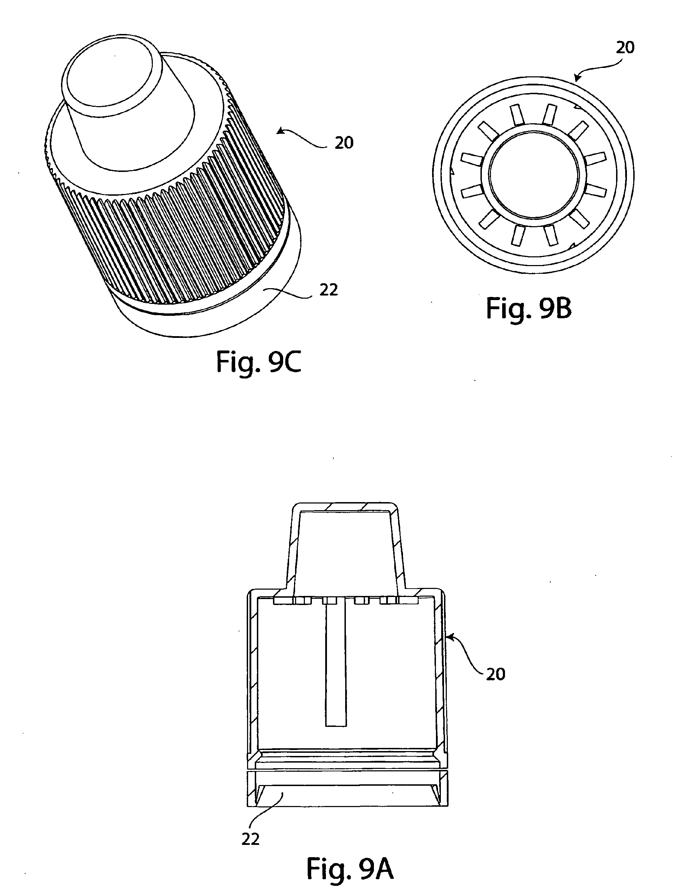

In order to complete the packaging and isolate the dropper from the exterior, the dispenser according to the invention is provided with a closure capsule (3) (visible in Figure 1 and, individually in Figure 3), which comprises two overlapping and cooperating elements. Such elements individually shown in Figures 8A-C and in Figures 9A-C consists of a screw cap (17) having the internal thread (18) complementary to the thread (19) provided on the neck of the closure element (4) (see Figure 3) and of an externally knurled overcap (20).

In the case of the device proposed according the invention, however, the capsule (3), or better, the cap (17) internal to said capsule (3) (which is shown without he corresponding the external overcap in Figure 10), also carries out the important function of cooperating with the elastomeric sleeve (9) in mechanically blocking the ports (8) provided on the intermediate portion (7) with the greater conicity of the dropper nozzle (5). Actually, the cylindrical projection (21) of the cap (17) (see, in particular Figures 3 and 8A) is shaped with suitable size for the cap (17), to abut with said projection, when the dispenser is closed, exactly on the intermediate portion (11) of the tubular sleeve (9), that is the portion which covers the intermediate portion (7) with greater conicity of the dropper nozzle (5), wherein the ports (8) for the passage of product from the interior of the bottle towards the exterior are provided.

The position of the cylindrical projection (21) of the cap (17), when the dispenser is closed, has the advantage of not mechanically interfering within elastomeric sleeve (9) present on the terminal portion (6) of the dropper nozzle (5), so that the said zone of the sleeve does not undergo any disturbance, damage or retraction due to the contact with the cap (17). On the contrary, the point of contact is displaced to a zone where the elastomeric sleeve (9) is less subject to stress and, if needed it could also be realized with greater thickness. In addition, the cooperation between the dropper nozzle (5) and the cylindrical projection (21) of the cap (17), right in the portion where the ports (8) for the passage of the product are present, guarantees against accidental leakage of the product which may originate from uncontrolled pressures on the container body (1).

In addition, the illustrated capsule device (3) comprises a particular mechanical coupling between the cap (17) and the external overcap (20) (see Figures 8A-C and 9A-C) which constitutes a safety closure device preventing the dispenser from being opened by children ("child-proof" device). The overcap (20) also comprises a tear-off removable collar (22) having function of guarantee seal (tamper-evident). The latter demonstrates, by being unbroken at the moment of the first opening, that dispenser is still sealed and has not been tampered with.

As it may be deduced from the above description, the dispenser according to the invention may be used in the same way as any normal dispenser for repeated administration of a product, and does not require any specific procedure for its use, but guarantees an immediate and automatic closure of the dropper when the user stops dispensing the product. This type of stable, safe, sterile and automatic closure occurs at the tip of the dropper, where the dropper nozzle is located and this guarantees the complete sterility of the product, its storage without any oxidative phenomena and its availability for repeated administrations up to the complete emptying of the bottle contents.

Moreover, the device is easy to use also by the most inexperienced or elderly users and allows to supply a pharmaceutical product in exactly metered drops, without giving rise to jets or sprays in the event that the dispenser is too energetically squeezed.

Finally, the structure of the proposed device is such that it is not excessively complex but it is extremely simple to produce from a manufacturing standpoint with a reduced number of pieces, some of which are the same as in the manufacturing lines already in use, for instance for the production of similar liquid products in bottles with the addition of preservatives. The dispenser according to the invention, therefore, appears to be extremely advantageous from the standpoint of the manufacturing flexibility of the corresponding apparatus.

The present invention has been disclosed with particular reference to some specific embodiments thereof, but it should be understood that modifications and changes may be made by the persons skilled in the art without departing from the scope of the invention as defined in the appended claims.

## Claims

1. A dispenser for liquid preparations without preservatives, comprising a container body (1), a dropper assembly (2), and a closure capsule (3) suitable to be coupled with said dropper assembly (2), wherein the dropper assembly (2), in turn, comprises a closure element (4) for the container body (1) ending with a rigid dropper nozzle (5) provided with one or more transversal passage ports (8), communicating with the interior of said container body covered with a resiliently deformable tubular sleeve (9), cooperating with said ports (8) for intercepting a liquid preparation contained in said dispenser, **characterized in that** the said dropper nozzle (5) comprises at least two axially subsequent portions having different conicity: a first portion with smaller conicity (6) making out the distal end of said dropper nozzle (5) and a second portion with greater conicity (7) on which said one or more ports (8) are provided, and **in that** said closure capsule comprises an internal projection (21) that comes into contact, when the capsule is closed, with said resiliently deformable tubular sleeve (9) at a position corresponding to the second portion of the dropper nozzle (5) with greater conicity (7), thereby blocking the possible passage of the liquid preparation through the said two or more ports (8).

2. A dispenser according to claim 1, wherein said said dropper assembly (2) further comprises a tubular protective element (14) coaxial with the said dropper nozzle (5), which extending outside said tubular sleeve (9) in such a way as to surround it for most of its length.

3. A dispenser according to claims 1 or 2, wherein said resiliently deformable tubular sleeve (9) comprises a first portion (10) and a second axially subsequent portion (11) and corresponding to said first and second portions of the said dispensing nozzle.

4. A dispenser according to claim 3, wherein said resiliently deformable tubular sleeve (9) comprises, at its end facing to the container body (1), a third portion (12), having a greater thickness than the previous ones, ending in an annular projection (13).

5. A dispenser according to claim 4, wherein said annular projection (13) is coupled with the end of the cited tubular protective element (14) facing towards the container body (1).

6. A dispenser according to claim 5, wherein said tubular protective element (14) is provided with an intermediate flange (15) which is fixedly blocked on said closure element (4) of the container body (1).

7. A dispenser according to anyone of claims 1-6, wherein the said two or more ports (8) provided on the said first portion with the greater conicity (7) of said dispensing nozzle (5) are three in number, placed at 120° one from the other around the periphery of the nozzle.

8. A dispenser according to anyone of claims 1-7, wherein the said container body (1), is a single-walled bottle type made of flexible resilient material or is of the double-walled type with a collapsible internal container and an air gap place there between, or it is of the single-walled collapsible tube type.

9. A dispenser according to claim 8, of the single-walled type made of flexible resilient material, wherein said dropper assembly (2) also comprises a venting valve (16) with a hydrophobic membrane for the passage of the gases only from and into the bottle.

10. A dispenser according to claim 9, wherein the said hydrophobic membrane has a porosity of 0.2 µm.

11. A dispenser according to claims 9 or 10, wherein said venting valve (16) with hydrophobic membrane is housed in a suitable seat within said closure element (4) in said container body (1).

12. A dispenser according to any one of claims 1-11, wherein said closure capsule (3) comprises a screw cap (17) suitable to protect said dropper assembly (2) and a knurled overcap (20) external to said screw cap (17) and connected thereto.

13. A dispenser according to claim 12, wherein the coupling between the said cap (17) and said overcap (20) creates a safety closure system preventing children from opening the bottle (child-proof device).

## Patentansprüche

1. Dispenser für Flüssigpräparationen ohne Konservierungsmittel, umfassend einen Behälterkörper (1), eine Tropfanordnung (2) und eine Schließkapsel (3), geeignet, um mit der Tropfanordnung (2) gekoppelt zu werden, wobei die Tropfanordnung (2) wiederum ein Schließelement (4) für den Behälterkörper (1) umfasst, endend mit einer steifen Tropfdüse (5), bereitgestellt mit einem oder mehreren Transversalpassageanschlüssen (8), kommunizierend mit dem Innern des Behälterkörpers, bedeckt mit einer elastisch verformbaren rohrförmigen Hülse (9), kooperierend mit den Anschlüssen (8) zum Aufnehmen einer Flüssigkeitspräparation, enthalten in dem Dispenser, **dadurch gekennzeichnet, dass** die Tropfdüse (5) zumindest zwei axial folgende Abschnitte umfasst mit unterschiedlicher Konizität: einen ersten Abschnitt mit kleinerer Konizität (6), ausbildend das distale Ende der Tropfdüse (5), und einen zweiten Abschnitt mit größerer Konizität (7), an dem der eine oder die mehreren Anschlüsse (8) vorgesehen sind, und dadurch, dass die Schließkapsel einen inneren Vorsprung (21) umfasst, der, wenn die Kapsel geschlossen ist, mit der elastisch verformbaren röhrenförmigen Hülse (9) in Kontakt kommt an einer Position entsprechend dem zweiten Abschnitt der Tropfdüse (5) mit größerer Konizität (7), wodurch die mögliche Passage der Flüssigkeitspräparation durch die zwei oder mehr Anschlüsse (8) geblockt wird.

2. Dispenser gemäß Anspruch 1, wobei die Tropfanordnung (2) ferner ein rohrförmiges Schutzelement (14) umfasst koaxial mit der Tropfdüse (5), sich erstreckend außenseitig der rohrförmigen Hülse (9), um sie für den Großteil ihrer Länge einzufassen.

3. Dispenser gemäß Anspruch 1 oder 2, wobei die elastisch verformbare rohrförmige Hülse (9) einen ersten Abschnitt (10) umfasst und einen zweiten axial folgenden Abschnitt (11) und entsprechend den ersten und zweiten Abschnitten der Dispensierdüse.

4. Dispenser gemäß Anspruch 3, wobei die elastisch verformbare rohrförmige Hülse (9) an ihrem Ende, zugewandt dem Behälterkörper (1), einen dritten Abschnitt (12) umfasst mit einer größeren Dicke als die Vorigen, endend in einem ringförmigen Vorsprung (13).

5. Dispenser gemäß Anspruch 4, wobei der ringförmige Vorsprung (13) gekoppelt ist mit dem Ende des genannten rohrförmigen Schutzelements (14), zugewandt dem Behälterkörper (1).

6. Dispenser gemäß Anspruch 5, wobei das rohrförmige Schutzelement (14) bereitgestellt ist mit einem Zwischenflansch (15), der fixiert geblockt ist an dem Schließelement (4) des Behälterkörpers (1).

7. Dispenser gemäß einem der Ansprüche 1-6, wobei die zwei oder mehr Anschlüsse (8), vorgesehen an dem ersten Abschnitt mit der größeren Konizität (7) der Dispensierdüse (5), drei an der Zahl sind, platziert um 120° voneinander um die Peripherie der Düse.

8. Dispenser gemäß einem der Ansprüche 1-7, wobei der Behälterkörper (1) ein einwandiger Flaschentyp ist, gemacht aus flexiblem, elastischem Material, oder ein doppelwandiger Typ ist mit einem kollabierbaren inneren Behälter und einer Luftspaltstelle dort dazwischen oder er ist ein einwandiger kollabierbarer Rohrtyp.

9. Dispenser gemäß Anspruch 8, gemäß dem einwandigen Typ, gemacht aus flexiblem, elastischen Material, wobei die Tropfanordnung (2) auch ein Lüftungsventil (16) umfasst mit einer hydrophobischen Membran für die Passage des Gases ausschließlich von und in die Flasche.

10. Dispenser gemäß Anspruch 9, wobei die hydrophobische Membran eine Porosität von 0,2 µm aufweist.

11. Dispenser gemäß Anspruch 9 oder 10, wobei das Lüftungsventil (16) mit hydrophobischer Membran beherbergt ist in einem geeigneten Sitz in dem Schließelement (4) in dem Behälterkörper (1).

12. Dispenser gemäß einem der Ansprüche 1-11, wobei die Schließkapsel (3) eine Schraubkappe (17) umfasst, geeignet, um die Tropfanordnung (2) zu schützen, und eine gerändelte Überkappe (20) extern zu der Schraubkappe (17) und damit verbunden.

13. Dispenser gemäß Anspruch 12, wobei die Kopplung zwischen der Kappe (17) und der Überkappe (20) ein Sicherheitsschließsystem erzeugt, das Kinder daran hindert, die Flasche zu öffnen (Kind-Sicherheits-Vorrichtung).

## Revendications

1. Distributeur de préparations liquides sans conservateurs, comprenant un corps de récipient (1), un ensemble compte-gouttes (2) et une capsule de fermeture (3) adaptée pour être couplée audit ensemble compte-gouttes (2), dans lequel l'ensemble compte-gouttes (2), à son tour, comprend un élément de fermeture (4) pour le corps de récipient (1) se terminant par une buse compte-gouttes rigide (5) équipée d'un ou plusieurs orifices de passage transversaux (8), communicant avec l'intérieur dudit corps de récipient recouvert d'un manchon tubulaire élastiquement déformable (9), coopérant avec lesdits orifices (8) pour intercepter une préparation liquide contenue dans ledit distributeur, **caractérisé en ce que** ladite buse compte-gouttes (5) comprend au moins deux parties axialement successives ayant une conicité différente : une première partie avec une conicité (6) plus petite constituant l'extrémité distale de ladite buse compte-gouttes (5) et une deuxième partie avec une conicité (7) plus grande sur laquelle lesdits un ou plusieurs orifices (8) sont prévus, et **en ce que** ladite capsule de fermeture comprend une saillie interne (21) qui vient en contact, lorsque la capsule est fermée, avec ledit manchon tubulaire élastiquement déformable (9) dans une position correspondant à la deuxième partie de la buse compte-gouttes (5) avec une conicité (7) plus grande, bloquant ainsi le passage éventuel de la préparation liquide à travers lesdits deux orifices (8) ou plus.

2. Distributeur selon la revendication 1, dans lequel ledit ensemble compte-gouttes (2) comprend en outre un élément protecteur tubulaire (14) coaxial avec ladite buse compte-gouttes (5), s'étendant vers l'extérieur dudit manchon tubulaire (9) de manière à l'entourer sur la majeure partie de sa longueur.

3. Distributeur selon les revendications 1 ou 2, dans lequel ledit manchon tubulaire élastiquement déformable (9) comprend une première partie (10) et une deuxième partie axialement successive (11) et correspondant auxdites première et deuxième parties de ladite buse de distribution.

4. Distributeur selon la revendication 3, dans lequel ledit manchon tubulaire élastiquement déformable (9) comprend, au niveau de son extrémité tournée vers le corps de récipient (1), une troisième partie (12), ayant une épaisseur supérieure aux deux précédentes, se terminant en une saillie annulaire (13).

5. Distributeur selon la revendication 4, dans lequel ladite saillie annulaire (13) est couplée à l'extrémité de l'élément protecteur tubulaire (14) cité tournée vers le corps de récipient (1).

6. Distributeur selon la revendication 5, dans lequel ledit élément protecteur tubulaire (14) est doté d'une bride intermédiaire (15) qui est bloquée à demeure sur ledit élément de fermeture (4) du corps de récipient (1).

7. Distributeur selon l'une quelconque des revendications 1 à 6, dans lequel lesdits deux orifices (8) ou plus prévus sur ladite première partie avec la conicité (7) plus grande de ladite buse de distribution (5) sont au nombre de trois, disposés à 120° les uns des autres autour de la périphérie de la buse.

8. Distributeur selon l'une quelconque des revendications 1 à 7, dans lequel ledit corps de récipient (1) est du type flacon monoparoi en matériau élastique souple ou est du type double paroi avec un récipient interne compressible et un intervalle d'air entre les deux, ou il est du type tube compressible monoparoi.

9. Distributeur selon la revendication 8, du type monoparoi en matériau élastique souple, dans lequel l'ensemble compte-gouttes (2) comprend également une valve d'aération (16) avec une membrane hydrophobe pour le passage des gaz provenant du flacon et entrant dans celui-ci uniquement.

10. Distributeur selon la revendication 9, dans lequel ladite membrane hydrophobe a une porosité de 0,2 µm.

11. Distributeur selon les revendications 9 ou 10, dans lequel ladite valve d'aération (16) avec une membrane hydrophobe est logée sur une surface d'appui adaptée à l'intérieur dudit élément de fermeture (4) dans ledit corps de récipient (1).

12. Distributeur selon l'une quelconque des revendications 1 à 11, dans lequel ladite capsule de fermeture (3) comprend un capuchon fileté (17) adapté pour protéger ledit ensemble compte-gouttes (2) et un surcapuchon moleté (20) à l'extérieur dudit capuchon fileté (17) et qui est relié à celui-ci.

13. Distributeur selon la revendication 12, dans lequel le couplage entre ledit capuchon (17) et ledit surcapuchon (20) crée un système de fermeture de sécurité empêchant les enfants d'ouvrir le flacon (dispositif de sécurité pour enfant).
